# EUROPEAN PATENT APPLICATION

(11) **EP 4 129 175 A1**
(43) Date of publication of application: **08.02.2023**
(21) Application number: 21781501.8
(22) Date of filing: 26.03.2021
(51) Int. Cl.: A61B 5/24, A61B 5/256

(54) **BIOSENSOR**

(30) Priority: 30.03.2020 JP 2020059654
(71) Applicant: Nitto Denko Corporation, Ibaraki-shi, Osaka 567-8680 (JP); Spchange, LLC, Yokohama-shi, Kanagawa 224-0032 (JP)
(72) Inventor: YOSHIOKA, Ryoma, Ibaraki-shi, Osaka 567-8680 (JP); KAMATA, Takatsugu, Yokohama-shi, Kanagawa 224-0032 (JP); HIRAI, Yusaku, Yokohama-shi, Kanagawa 224-0032 (JP); UEDA, Masayuki, Yokohama-shi, Kanagawa 224-0032 (JP)
(74) Representative: Hoffmann Eitle
(86) International application number: PCT/JP2021/013154
(87) International publication number: WO 2021/200765

(57) **Abstract**

A living body sensor includes a flexible substrate that includes a body section; a first pad section provided via a first constricted section at one end in a longitudinal direction of the body section and including a first electrode configured to be attached to a living body; a second pad section provided via a second constricted section at another end in the longitudinal direction of the body section and including a second electrode configured to be attached to the living body; an obtaining section configured to obtain biological information through the first electrode and the second electrode; a wireless communication section configured to transmit the biological information obtained by the obtaining section; a component mounting section provided at the first constricted section side of the body section; and a battery setting section provided at the second constricted section side of the body section, a battery for supplying power to the component mounting section being set at the battery setting section. The body section, the first pad section, the second pad section, the obtaining section, the wireless communication section, the component mounting section, and the battery setting section are formed integrally with the flexible substrate. This allows deformation of the living body sensor in accordance with deformation caused by a movement of the living body, thereby providing the living body sensor that can reduce discomfort felt by the living body while the living body sensor is attached to the living body.

## Description

### [Technical Field]

The present invention relates to a living body sensor.

### [Background Art]

A wearable living body sensor is known that can be attached to a living body to obtain biological information such as an electrocardiographic signal over a long time. For example, a living body sensor of this type has electrodes on both longitudinal directional sides, the electrodes are affixed to a chest of a living body with the longitudinal direction aligned with a sternum, and then, the living body sensor automatically starts measuring biological information (see, for example, Patent Document 1) .

### [Prior Art Documents]

### [Patent Documents]

[Patent Document 1] the specification of U.S. Patent Application Publication No. 2019/0254553

### [Summary of Invention]

### [Problem to be Solved by Invention]

When biological information is obtained for a long time by a living body sensor attached to a living body, measurement for a long time becomes useless if the living body sensor cannot obtain biological information properly. In order to correctly obtain biological information for a long time, it is necessary that the living body sensor continues to obtain biological information without being dropped off the living body. In addition, in order to prevent the living body sensor from peeling off the living body, it is preferable that the living body sensor deforms in accordance with a deformation of a body surface of the living body caused by a body movement of the living body, and at the same time, it is preferable that discomfort felt by the living body on which the living body sensor is attached can be reduced.

The present invention has been devised in view of the above-described points, and the present invention has an object to provide a living body sensor that can deform in accordance with a deformation of a body surface of the living body caused by a body movement of the living body, thereby reducing discomfort felt by the living body while the living body sensor is attached to the living body.

### [Means for Solving Problem]

A living body sensor according to an embodiment of the present invention includes a flexible substrate that includes a body section; a first pad section provided at one end in a longitudinal direction of the body section via a first constricted section and including a first electrode configured to be affixed to a living body; a second pad section provided at another end in the longitudinal direction of the body section via a second constricted section and including a second electrode configured to be affixed to the living body; an obtaining section configured to obtain biological information via the first electrode and the second electrode; a wireless communication section configured to transmit the biological information obtained by the obtaining section; a component mounting section provided at the first constricted section side of the body section; and a battery setting section provided at the second constricted section side of the body section, a battery for supplying power to the component mounting section being set at the battery setting section. The body section, the first pad section, the second pad section, the obtaining section, the wireless communication section, the component mounting section, and the battery setting section are formed integrally with the flexible substrate.

### [Advantageous Effects of Invention]

According to the disclosed technique, it is possible to provide a living body sensor that can deform in accordance with a deformation of a body surface of a living body due to a body movement of the living body. In addition, it is possible to reduce discomfort felt by the living body while the living body sensor is attached to the living body.

### [Brief Description of Drawings]

Fig. 1 is a diagram illustrating an example of a whole configuration of a living body sensor system including a living body sensor according to an embodiment.
Fig. 2 is a layout diagram illustrating an example of a flexible substrate depicted in Fig. 1.
Fig. 3 is a diagram illustrating a state in which the living body sensor depicted in Fig. 1 is affixed to a chest of a subject.
Fig. 4 is a state transition diagram depicting an example of a transition of a mode of operation of the living body sensor depicted in Fig. 1.

### [Mode for Carrying out Invention]

Hereinafter, embodiments of the invention will be described with reference to the drawings. In each drawing, the same components are indicated by the same reference numerals and overlapping descriptions may be omitted.

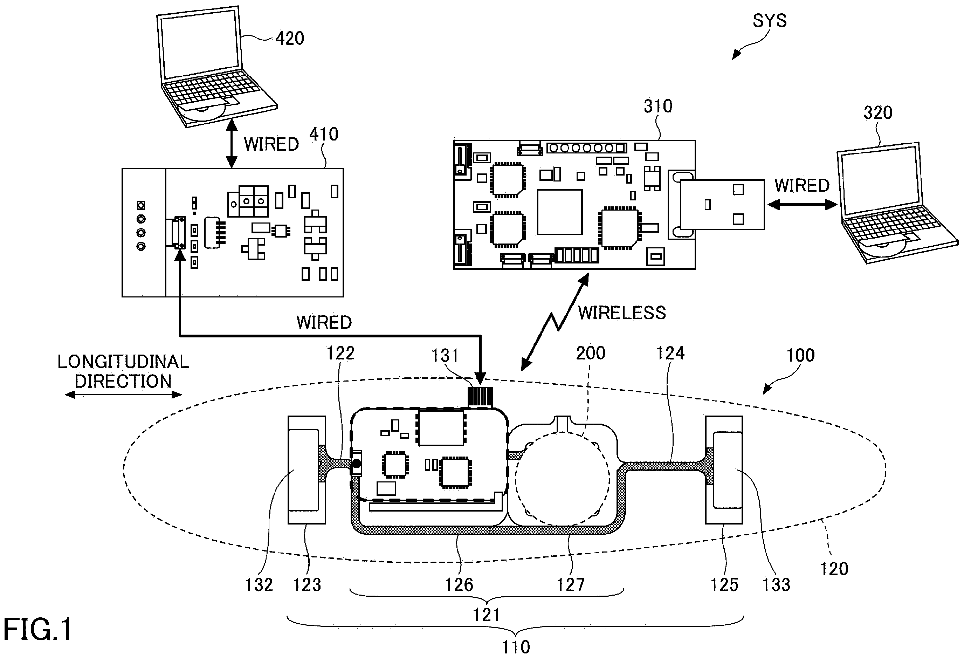

Fig. 1 is a diagram illustrating an example of a whole configuration of a living body sensor system including a living body sensor according to an embodiment. The living body sensor system SYS depicted in Fig. 1 includes a living body sensor 100, an operation checking device 310 for checking an initial operation, a personal computer (PC) 320, a reading device 410 for reading biological information from the living body sensor 100, and a PC 420. The living body sensor 100 is, for example, a wearable electrocardiograph that obtains an electrocardiographic signal from a living body. The living body sensor 100 may have a function of obtaining biological information other than an electrocardiographic signal, and may have a function of obtaining plural sorts of biological information.

The operation checking device 310 is connected to the PC 320 via, for example, a universal serial bus (USB) interface (wired). The operation checking device 310 has a capability of wirelessly communicating with the living body sensor 100 under the control of the PC 320. For example, the PC 320 has a function of displaying a waveform representing a time change in received biological information (e.g., an electrocardiographic waveform) on a display screen.

The reading device 410 is connected to the PC 420, for example, via a USB interface (wired). The reading device 410 has a function of communicating with the living body sensor 100 via a communication cable (wired communication).

The living body sensor 100 includes a flexible substrate (a resin substrate) 110 and a housing 120 (depicted by a dashed line) on which various components are mounted for obtaining biological information and processing the obtained biological information. The flexible substrate 110 has a body section 121, a constricted section 122 provided at one longitudinal directional end of the body section 121, and a pad section 123 connected to the body section 121 via the constricted section 122. The flexible substrate 110 also includes a constricted section 124 provided at the other longitudinal directional end of the body section 121 and a pad section 125 connected to the body section 121 via the constricted section 124.

The body section 121, the constricted section 122, the pad section 123, the constricted section 124, and the pad section 125 are integrally formed. By thus integrally forming these elements of the living body sensor 100 using the flexible substrate 110, assembly costs of the living body sensor 100 can be reduced and manufacturing costs of the living body sensor 100 can be reduced as compared to assembling a living body sensor 100 by combining separate elements.

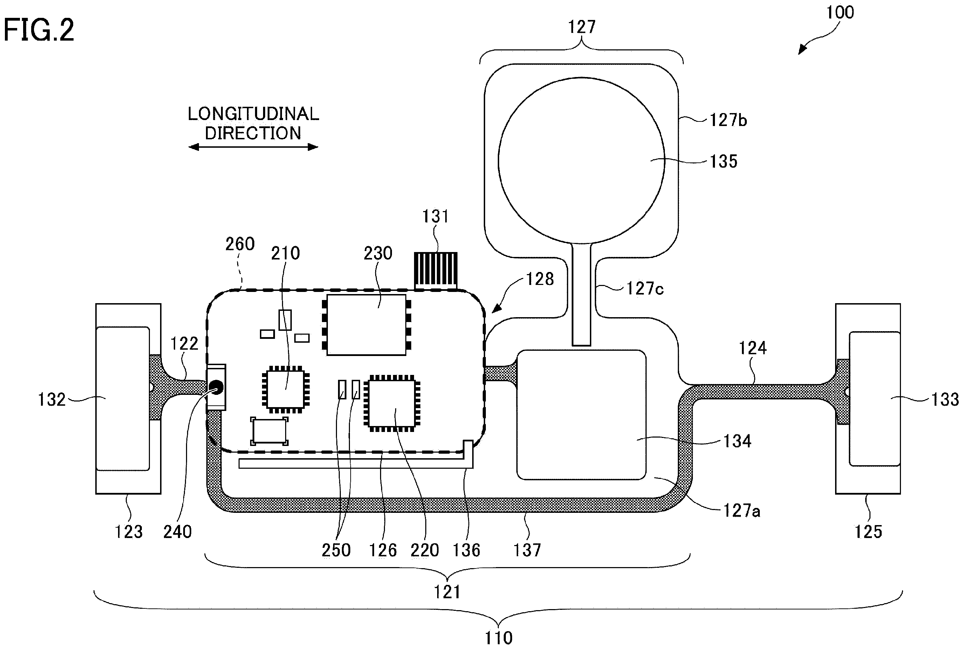

The body section 121 includes a component mounting section 126 at the constricted section 122 side and a battery setting section 127, to which a battery 200 is set, at the constricted section 124 side. An external terminal 131, to which a connector of a communication cable to be connected to the reading device 410 is connected, is formed in the component mounting section 126. Main components mounted on the component mounting section 126 will be described with reference to Fig. 2.

For example, a coin-type battery 200 that supplies power to the component mounting section 126 is set at the battery setting section 127. An electrode pattern 132 is formed on the pad section 123 to be affixed to a body surface of a living body, and an electrode pattern 133 is formed on the pad section 125 to be affixed to the body surface of the living body. Hereinafter, the electrode pattern 132 is also referred to as an electrode 132, and the electrode pattern 133 is also referred to as an electrode 133.

Fig. 2 is a layout diagram illustrating an example of the flexible substrate 110 depicted in Fig. 1. On the component mounting section 126 of the flexible substrate 110, an application specific integrated circuit (ASIC) 210, a system on a chip (SoC) 220, a flash memory 230, a switch 240, and light emitting diodes (LED) 250 are mounted.

Although being not particularly limited, the flash memory 230 is of a NAND type, for example. A LED that outputs green light and a LED that outputs red light are mounted to the component mounting section 126 as the LED 250. Hereinafter, the LED outputting green light is also referred to as a LED(G) and the LED outputting red light is also referred to as a LED(R).

The living body sensor 100 includes a plate member 260 (depicted in Fig. 2 as a frame drawn by a thick broken line), such as a stainless steel plate, on a surface (back side), included in the flexible substrate 110, opposite to a component mounting surface (front side) on which the components such as the ASIC 210 and the SoC 220 are mounted. The plate member 260 prevents the component mounting section 126 from being warped when the flexible substrate 110 is warped due to a change in a posture of a living body to which the living body sensor 100 is affixed. Thus, it is possible to prevent a disconnection of the wiring pattern due to the flexible substrate 110 being warped, and a portion at which a terminal of an electronic component such as the ASIC 210 is soldered to a pattern on the flexible substrate 110 from being damaged.

The switch 240 is, for example, a depression switch that is set to an ON state when a protrusion is depressed and set to an OFF state when the protrusion is not depressed. The switch 240 is mounted at a position that is next to the constricted section 122 (at an edge of the body section 121) and is opposite the plate member 260. As a result, when the switch 240 is depressed while the living body sensor 100 is attached to a living body, stress caused by the depressing is applied to an edge of the component mounting section 126. Therefore, warping of the component mounting section 126 (the plate member 260) occurring due to depressing of the switch 240 can be minimized, and the wiring pattern of the component mounting section 126 can be prevented from being broken or the like. Hereinafter, a living body to which the living body sensor 100 is affixed and from which biological information is obtained by the living body sensor 100 is also referred to as a subject P.

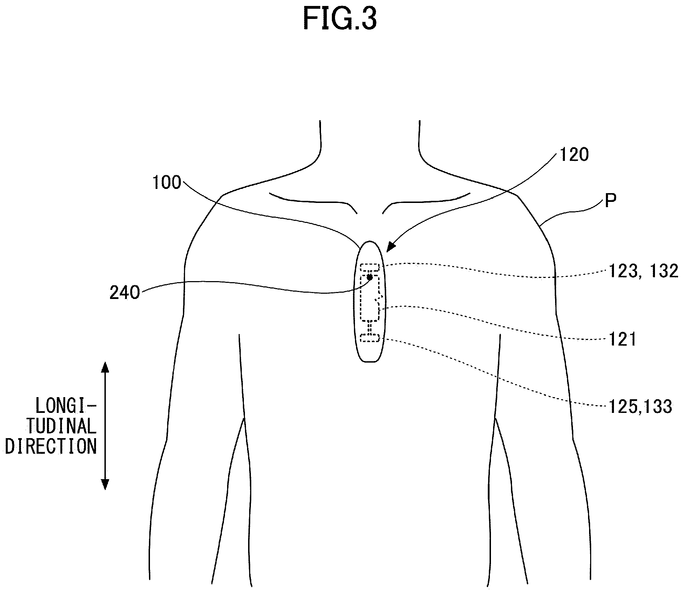

The constricted section 124 is formed to be longer than the constricted section 122. As illustrated in Fig. 3, the living body sensor 100 is affixed along a sternum of a subject P, while the pad section 123 is faced upward (i.e., toward the subject P neck side). At this time, the pad section 125 may be located at a lower edge of the sternum (near a stomach) of the subject P whose body height is small. However, the constricted section 124 that is long and narrow allows the constricted section 124 to be deformed in accordance with bending when the subject P bends his or her body, thereby reducing discomfort felt by the subject P to which the living body sensor 100 is attached. Also, because the constricted section 124 deforms in response to bending of the body of the subject P, it is possible to reduce a possibility that the electrodes 132 and 133 adhering to the body surface of the subject P peel off the body surface due to the bending of the body of the subject P.

Adhesion of electrodes 132 and 133 to a body surface of a subject P may be implemented with the use of an electrically conductive adhesive; or may be implemented with the use of a non-electrically-conductive adhesive to be applied to a part of each of the electrodes 132 and 133. Alternatively, an electrode that is specially prepared for being affixed to a living body and is affixed to each of the electrodes 132, and 133 may be used to affix the electrodes 132, and 133 to the subject P with the use of an electrically conductive adhesive or a non-electrically-conductive adhesive.

The battery setting section 127 includes pad sections 127a and 127b and a constricted section 127c. The pad section 127a is provided between the constricted section 124 and the component mounting section 126. The pad section 127b is provided, in a direction orthogonal to the longitudinal direction, with respect to the pad section 127a (an upper direction of Fig. 2) at a predetermined distance from the pad section 127a. The constricted section 127c is provided between pad sections 127a and 127b to connect the pad sections 127a and 127b together.

The pad section 127a has a positive electrode pattern 134 to which a positive terminal of a battery 200 (Fig. 1) is connected. The pad section 127b has a negative electrode pattern 135 to which a negative terminal of the battery 200 is connected. For example, the positive electrode pattern 134 has a square shape with corners chamfered, and the negative electrode pattern 135 has a circular shape corresponding to a size of a circular shape of the negative terminal of the battery 200. For example, a diameter of the negative electrode pattern 135 is equal to a diameter of the battery 200 and is equal to a length of a diagonal of the positive electrode pattern 134.

When a battery 200 is set in the living body sensor 100, an electrically conductive adhesive, such as an adhesive tape, is attached throughout the positive and negative electrode patterns 134 and 135. Next, for example, the positive terminal of the battery 200 is adhered to the positive electrode pattern 134. The constricted section 127c is then bent so that the pad sections 127a and 127b face each other, and the negative electrode pattern 135 is adhered to the battery 200 in a manner of being coincident in position with the circular shape of the negative terminal of the battery 200.

Alternatively, when a battery 200 is set in the living body sensor 100, the negative terminal of the battery 200 is adhered to the negative electrode pattern 135 via an adhesive, in such a manner that an outer periphery of the negative terminal of the battery 200 is coincident in position with an outer periphery of the negative electrode pattern 135. Thereafter, the constricted section 127c is bent so that the pad sections 127a, 127b face each other, and the positive terminal of the battery 200 is adhered to the positive electrode pattern 134 via an adhesive. The body section 121 depicted in Fig. 1 is in a state where a battery 200 is set at the battery setting section 127 with the battery 200 sandwiched between the positive electrode pattern 134 and the negative electrode pattern 135 with the constricted section 127c bent.

Making the shape of the negative electrode pattern 135 to be the same as the shape of the negative terminal of a battery 200 prevents the negative electrode pattern 135 from being short-circuited with a side surface (the positive terminal) of the battery 200. Further, adhering the negative electrode pattern 135 to the negative terminal of a battery 200 in a manner of being coincident in position with the circular shape of the negative terminal of the battery 200 prevents the negative terminal of the battery 200 from protruding from the periphery of the negative electrode pattern 135. Thus, it is possible to prevent the negative electrode pattern 135 from being short-circuited with the side surface of the battery 200 even if a posture of a subject P to which the living body sensor 100 is attached changes.

A battery 200 is adhered to the positive electrode pattern 134 and the negative electrode pattern 135 in a surface-to-surface contact manner via an electrically conductive adhesive. This reduces contact resistance compared to a case of connecting a battery 200 to the battery setting section 127, for example, via terminals, such as terminals of a leaf spring type. In addition, even when a subject P twists his or her body or the like, it is possible to prevent a battery 200 from being removed from the battery setting section 127, by adhering the battery 200 to the electrodes 134 and 135 in a surface-to-surface contact manner via an electrically conductive adhesive.

In addition, by thus sandwiching a battery 200 by the battery setting section 127 that is integrally formed onto the flexible substrate 110 and thus setting the battery 200 to the battery setting section, a thickness of the living body sensor 100 can be reduced compared to a case where a battery holder that is a separate body with respect to the flexible substrate 110 is used. This minimizes a protruding amount of the living body sensor 100 from a body surface of a subject P when the living body sensor 100 is affixed to the subject P. As a result, discomfort felt by the subject P with the living body sensor 100 attached to the subject P can be minimized.

By integrally forming the living body sensor 100 using the flexible substrate 110, the living body sensor 100 can be reduced in its weight as compared to a case where a plurality of components are brought together to form a living body sensor 100. Therefore, discomfort felt by a subject P to which the living body sensor 100 is attached can be further reduced and a possibility of the living body sensor 100 peeling off the subject P due to gravity can be reduced.

The flexible substrate 110 has an antenna pattern 136 formed along the longitudinal direction of the flexible substrate 110 near one (at a lower side in Fig. 2) of four sides of the rectangular component mounting section 126. Although not depicted, one end of the antenna pattern 136 is connected to the SoC 220. The flexible substrate 110 also has a wiring pattern 137 formed at an edge (at a lower side in Fig. 2) of the body section 121 and extending from the electrode pattern 133 to near the switch 240 through the constricted section 124. The wiring pattern 137 connects the electrode 133 to the ASIC 210.

The ASIC 210 is also connected to the electrode 132, obtains biological information from a subject P through the electrodes 132 and 133, and outputs the obtained biological information to the SoC 220. The ASIC 210 is an example of an obtaining section. The SoC 220 has a wireless communication section that wirelessly communicates with the operation checking device 310 and transmits the biological information received from the ASIC 210 to the operation checking device 310 during an operation checking mode, which will be described later. That is, the SoC 220 functions as a wireless communication section. The SoC 220 writes the biological information received from the ASIC 210 to the flash memory 230 during a biological information recording mode, which will be described later.

The operation checking mode is a mode of checking as to whether the living body sensor 100 can properly obtain biological information (whether the living body sensor 100 is properly attached to the subject P and whether the living body sensor 100 operates normally). In the operation checking mode, the SoC 220 transmits biological information received from the ASIC 210 to the operation checking device 310 depicted in Fig. 1 via the internal wireless communication section without writing the biological information to the flash memory 230.

The biological information recording mode is a mode of operation switched from the operation checking mode based on a recording start instruction input from the operation checking device 310 when it is checked in the operation checking mode that biological information can be properly obtained by the living body sensor 100. During the biological information recording mode, the SoC 220 sequentially writes biological information obtained from the ASIC 210 to the flash memory 230.

The antenna pattern 136 is formed at a wiring layer of the flexible substrate 110 at a component mounting surface side (the front side). Meanwhile, the wiring pattern 137 is formed at a wiring layer of the flexible substrate 110 at the back side. This prevents a DC current flowing through the wiring pattern 137 from flowing through the antenna pattern 136 even when, for example, the electrode pattern 133 contacts a charged object and a discharge toward the electrode pattern 133 occurs. Accordingly, the DC current flowing out due to the discharge can be prevented from flowing through the antenna pattern 136 into the SoC 220, and thus, elements in the SoC 220 can be prevented from being electrostatically destroyed. In particular, it is possible to avoid a damage to the wireless communication section connected to the antenna pattern 136. The ASIC 210 includes a protective device against electrostatic discharge at an area where an input circuit to which the wiring pattern 137 is connected is formed.

The flexible substrate 110 has a slit 128 extending toward the inside along a direction perpendicular to the longitudinal direction from an edge between the component mounting section 126 and the battery setting section 127. By providing the slit 128, the flexible substrate 110 can be bent at a position of the slit 128 if the flexible substrate 110 is stressed due to a change in a posture of a subject P to which the living body sensor 100 is affixed. Accordingly, the body section 121 can be warped at the position of the slit 128 in accordance with a deformation of a body surface of a subject P caused by a movement of the subject P, thereby enabling reduction of discomfort felt by the subject P with the living body sensor 100 attached to the subject P.

Fig. 3 is a diagram illustrating a state in which the living body sensor 100 depicted in Fig. 1 is affixed to a chest of a subject P. For example, the living body sensor 100 is affixed to the subject P with the pad section 123 at an upper side and the pad section 125 at a lower side, with the longitudinal direction of the living body sensor 100 extending along a sternum of the subject P. That is, the living body sensor 100 is affixed to the subject P with the longer constricted section 124 at the lower side. On the back side of the body section 121 of the living body sensor 100, an adhesive tape or an adhesive agent is provided to affix the body section 121 to a body surface of the subject P.

The housing 120 of the living body sensor 100, with the body section 121 contained therein, has openings at least at positions corresponding to the electrodes 132, 133. The electrodes 132, 133 exposed from the openings can be adhered to the subject P. The living body sensor 100 wirelessly communicates with the operation checking device 310 (Fig. 1) in a state in which the living body sensor 100 has been affixed to the subject P, and the electrodes 132 and 133 have been adhered to a body surface of the subject P. The living body sensor 100 transmits biological information, such as an electrocardiographic signal obtained from the subject P, to the PC 320 (see Fig. 1) via the operation checking device 310.

Subsequently, based on an electrocardiographic waveform or the like displayed on a display screen of the PC 320, a physician or the like determines that the living body sensor 100 has been affixed to a proper position. The living body sensor 100 then starts an actual measurement of biological information in response to a recording start command transmitted from the PC 320 via the operation checking device 310 based on an operation of the PC 320 performed by the physician or the like.

The living body sensor 100 writes biological information, which has been obtained sequentially from the subject P during the measurement, together with time information, to the flash memory 230. When the switch 240 is depressed during the measurement, and then, the switch 240 is kept continuously in the turned-on state, the living body sensor 100 sequentially writes time information (indicating the turned-on state) corresponding to the current time to the flash memory 230.

The subject P with the living body sensor 100 attached thereto depresses the switch 240 if the subject P feels ill such as a palpitation or shortness of breath. While the subject P is feeling ill, the subject P may depress the switch 240 continuously. After the measurement is completed, the reading device 410 reads biological information, such as electrocardiographic signal data, time counter information accompanied by the biological information, and time counter information indicative of turned-on states of the switch 240, for example, from the flash memory 230 of the living body sensor 100.

The reading device 410 (Fig. 1) transfers various information read from the flash memory 230 to the PC 420 (Fig. 1). The PC 420 that receives the various information displays biological information, such as an electrocardiographic waveform, on the display screen, and displays timings when the switch 240 has been depressed. This allows the physician or the like operating the PC 420 to determine whether abnormality is present in an electrocardiographic waveform or the like obtained when the subject P felt ill.

For example, a duration of the measurement is set according to a duration for which the living body sensor 100 is operable by power supply from the battery 200. For example, the duration of the measurement may be 24 hours (1 day), but may be longer depending on the capacity of the battery 200 and the power consumption of the living body sensor 100.

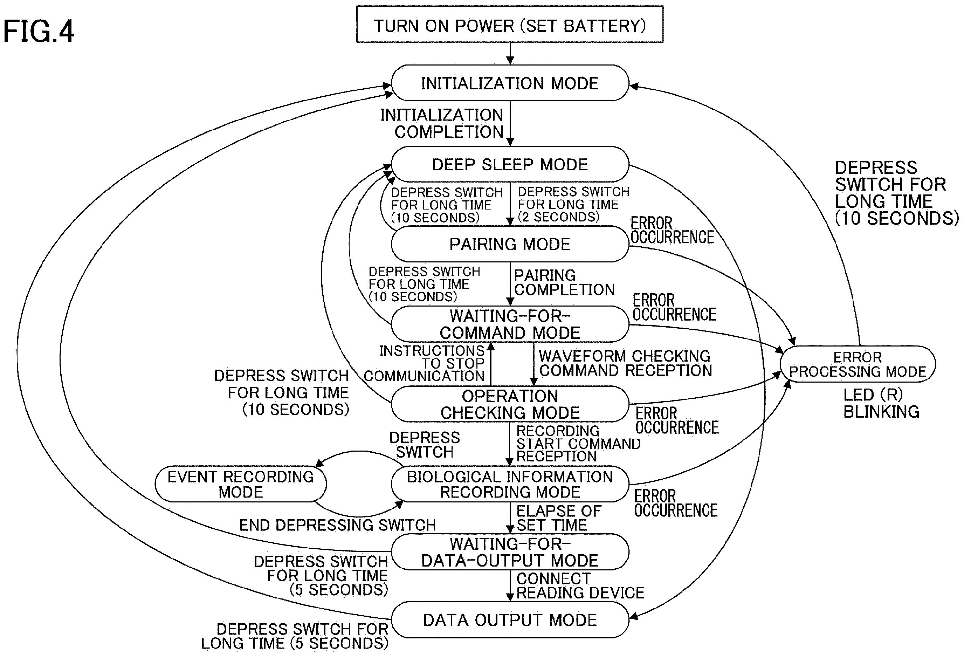

Fig. 4 is a state transition diagram illustrating an example of transitions of modes of operations of the living body sensor 100 depicted Fig. 1. For example, the state transitions depicted in Fig. 4 are implemented when a MCU built in the SoC 220 executes a control program. The control program executed by the SoC 220 is a program for controlling the overall operation of the living body sensor 100.

The living body sensor 100 transitions to an initialization mode when the battery 200 is set at the battery setting section 127 and supply of power to the living body sensor 100 is initiated. The living body sensor 100 performs initial settings of the hardware and the like in the initialization mode. After the completion of initialization, the operation mode transitions to a deep sleep mode. The deep sleep mode is a mode in which the living body sensor 100 receives an interrupt caused by the switch 240 being depressed, and wireless communication functions of communicating with the ASIC 210 and the operation checking device 310 are deactivated in the mode.

In the deep sleep mode, the living body sensor 100 transitions to a pairing mode when depression of the switch 240 for a long time (e.g., 2 seconds) is detected, and maintains the deep sleep mode in a case where a duration of the switch 240 being depressed is shorter than 2 seconds. In the deep sleep mode, the living body sensor 100 transitions to a data output mode when the reading device 410 is connected to the external terminal.

In the pairing mode, the living body sensor 100 causes the wireless communication section of the SoC 220 to perform pairing with the operation checking device 310. When the pairing is completed, the living body sensor 100 transitions to a waiting-for-command mode. If an error occurs during the pairing, the living body sensor 100 transitions to an error processing mode and performs error processing. The living body sensor 100 causes the LED(R) to blink in a predetermined pattern (e.g., at 1 second intervals) while the living body sensor 100 is in the error processing mode.

In the waiting-for-command mode, the living body sensor 100 transitions to an operation checking mode when a waveform checking command is received from the PC 320 via the operation checking device 310. If an error occurs in the waiting-for-command mode, the living body sensor 100 transitions to the error processing mode.

In the operation checking mode, the living body sensor 100 sends instructions to the ASIC 210 to obtain biological information, and sequentially receives biological information obtained by the ASIC 210. The living body sensor 100 transmits the received biological information to the PC 320 via the operation checking device 310. When receiving instructions, to stop communication, from the PC 320 via the operation checking device 310 during the operation checking mode, the living body sensor 100 causes the ASIC 210 to stop obtaining biological information, and returns to the waiting-for-command mode. If an error occurs in the operation checking mode, the living body sensor 100 transitions to the error processing mode.

In the operation checking mode, when a recording start command is received from the PC 320 via the operation checking device 310, the living body sensor 100 transitions to a biological information recording mode. Upon the transition from the operation checking mode to the biological information recording mode, obtaining of biological information by the ASIC 210 continues, for example. It is noted that, when the switch 240 is depressed for a long time (for example, 10 seconds) in each of the pairing mode, the waiting-for-command mode, the operation checking mode, and the error processing mode, the operation mode returns to the deep sleep mode.

In the biological information recording mode, the living body sensor 100 sequentially writes biological information received from the ASIC 210 to the flash memory 230. When the switch is depressed in the biological information recording mode, the living body sensor 100 transitions to an event recording mode and then continues to be in the event recording mode until the switch 240 comes to be in a turned-off state. In the event recording mode, the living body sensor 100 sequentially writes biological information received from the ASIC 210 and time information to the flash memory 230. That is, operations performed in the event recording mode are a duplicate of operations performed in the biological information recording mode.

When a predetermined set time has elapsed (e.g., 24 hours) in the biological information recording mode, the living body sensor 100 sends instructions to the ASIC 210 to stop obtaining biological information, and transitions to a waiting-for-data-output mode. In the waiting-for-data-output mode, the living body sensor 100 waits for the reading device 410 to be connected to the external terminal 131. When the reading device 410 has been connected to the external terminal 131, the living body sensor 100 transitions to a data output mode. In the data output mode, the reading device 410 accesses the flash memory 230 through the external terminal 131 to read biological information, time information, and the like stored in the flash memory 230. In each of the waiting-for-data-output mode and the data output mode, if the switch 240 is depressed for a long time (e.g., 5 seconds), the operation mode returns to the initialization mode, and initial settings are performed in the initialization mode.

As depicted in Fig. 4, an operation mode of the living body sensor 100 may be transitioned to another operation mode from among various operation modes, depending on when the switch 240 is depressed and an operation mode of the living body sensor 100 at the time when the switch 240 is depressed. Therefore, as described above, the single switch 240 can implement soft switches with which it is possible to detect a plurality of events. As a result, because only the single switch 240 is sufficient to be mounted to the living body sensor 100, the living body sensor 100 can be miniaturized and the cost of the living body sensor 100 can be reduced.

As described above, in the embodiment depicted in Figs. 1-4, each element of the living body sensor 100 can be integrally formed via the flexible substrate 110 to make the living body sensor 100 simpler in structure than a case of assembling a living body sensor 100 by combining separate components. This can reduce the manufacturing costs and the assembly costs of the living body sensor 100.

By integrally forming the living body sensor 100 via the flexible substrate 110, the living body sensor 100 affixed to a subject P can be deformed in accordance with a deformation of a body surface of the subject P caused by a movement of the subject P. Accordingly, it is possible to avoid a failure such as breaking of a wiring pattern due to warping of the flexible substrate 110. In addition, it is possible to reduce a possibility that the electrodes 132 and 133 adhering to the subject P peel off from the body surface due to bending, thereby reducing discomfort felt by the subject P while the living body sensor 100 is attached to the subject P.

It is possible to prevent an electrostatic current present in the pad sections 123 and 125 from flowing into the antenna pattern 136 and damaging the antenna pattern 136. Further, it is possible to prevent an electric current from flowing into the component mounting section 126 through the antenna pattern 136 and it is possible to avoid an electrostatic discharge damage of an electronic component such as the SoC 220 mounted on the component mounting section 126.

Because the battery setting section 127 can be integrally formed with the flexible substrate 110, the living body sensor 100 can be simplified in structure and manufacturing costs can be reduced. In addition, because a thickness of the living body sensor 100 can be thus reduced, a protruding amount of the living body sensor 100 when the living body sensor 100 is affixed to a subject P can be minimized. Further, because all the elements other than the components mounted on the component mounting section 126 can be formed by electrically conductive patterns, manufacturing costs and assembly costs can be reduced.

The negative electrode pattern 135 is formed to correspond to the circular shape of the negative terminal of the battery 200. Therefore, when the negative terminal of the battery 200 is adhered to the negative electrode pattern 135 in a manner of being coincident in position with the negative electrode pattern 135, it is possible to prevent the periphery of the negative terminal of the battery 200 from extending from the periphery of the negative electrode pattern 135. Thus, it is possible to prevent the negative electrode pattern 135 from short-circuiting with the side wall of the battery 200 even if a posture of a subject P to which the living body sensor 100 is attached changes in any way.

The battery 200 is adhered to the positive electrode pattern 134 and the negative electrode pattern 135 in a surface-to-surface contact manner via a conductive adhesive. This reduces the contact resistance and prevents the battery 200 from being removed from the battery setting section 127, as compared to a case where the battery 200 were set at the battery setting section 127 via terminals, such as terminals of leaf springs.

Further, by sandwiching the battery 200 by the battery setting section 127 to set it to the battery setting section 127, that is integrally formed with the flexible substrate 110, the thickness of the living body sensor 100 can be reduced compared to a case of using a flexible substrate 110 and a battery holder that is a separate body with respect to the flexible substrate 110. This minimizes the protruding amount of the living body sensor 100 from a body surface of a subject P when the living body sensor 100 is attached to the subject P. As a result, discomfort felt by the subject P with the living body sensor 100 attached thereto can be minimized.

The slit 128 allows the body section 121 to warp around the slit 128 in accordance with a deformation of a body surface of a living body caused by a body movement while the living body sensor 100 is attached to a subject P, thereby reducing a discomfort felt by the subject P while the living body sensor 100 is attached to the subject P. It is possible to release stress applied to the body section 121 due to a deformation of a body surface of a living body as a result of a portion surrounding the slit 128 warping, and it is possible to prevent the stress from being applied to the component mounting section 126. Accordingly, it is possible to prevent the wiring pattern or the like of the component mounting section 126 from being damaged.

Rigidity of the plate member 260 can reduce warping of the component mounting section 126 and prevent the wiring pattern of the component mounting section 126 from being broken. Warping of the component mounting section 126 can be reduced because stress generated when the switch 240 is depressed is applied to an edge of the component mounting section 126.

Although the present invention has been described based on the embodiments, the present invention is not limited to the specifically disclosed embodiments. In these respects, modifications/changes can be made without departing from the spirit of the present invention.

The present application claims priority to Japanese patent application No. 2020-059654 filed March 30, 2020, and the entire contents of Japanese patent application No. 2020-059654 are herein incorporated by reference.

### [Description of Symbols]

100 Living body sensor
110 Flexible substrate
120 Housing
121 Body section
122 Constricted section
123 Pad section
124 Constricted section
125 Pad section
126 Component mounting section
127 Battery setting section
127a, 127b Pad sections
127c Constricted section
128 Slit
131 External terminal
132, 133 Electrode patterns
134 Positive electrode pattern
135 Negative electrode pattern
136 Antenna pattern
200 Battery
210 ASIC
220 SoC
230 Flash memory
240 Switch
250 LED
260 Plate member
310 Operation checking device
320 PC
410 Reading device
420 PC
P Subject
SYS Living body sensor system

## Claims

1. A living body sensor, comprising a flexible substrate that includes:
a body section;
a first pad section provided at one end in a longitudinal direction of the body section via a first constricted section and including a first electrode configured to be attached to a living body;
a second pad section provided at another end in the longitudinal direction of the body section via a second constricted section and including a second electrode configured to be attached to the living body;
an obtaining section configured to obtain biological information through the first electrode and the second electrode;
a wireless communication section configured to transmit the biological information obtained by the obtaining section;
a component mounting section provided at the first constricted section side of the body section; and
a battery setting section provided at the second constricted section side of the body section, a battery for supplying power to the component mounting section being set at the battery setting section,
wherein
the body section, the first pad section, the second pad section, the obtaining section, the wireless communication section, the component mounting section, and the battery setting section are formed integrally with the flexible substrate.

2. The living body sensor as claimed in claim 1,
wherein
the flexible substrate includes an antenna pattern provided along the longitudinal direction of the body section and connected to the wireless communication section at a surface opposite to a surface at which a wiring pattern that connects the second pad section and the obtaining section is formed.

3. The living body sensor as claimed in claim 1 or 2,
wherein
the battery setting section includes:
a first electrode pattern provided next to the component mounting section and configured to be connected to a first terminal of the battery placed at the battery setting section; and
a second electrode pattern provided via a third constricted section in a direction with respect to the first electrode pattern, the direction being perpendicular to the longitudinal direction,
wherein
when the third constricted section is bent, the second electrode pattern faces the first electrode pattern and is connected to a second terminal of the battery.

4. The living body sensor as claimed in claim 3, further comprising:
an electrically conductive first adhesive provided on the first electrode pattern; and
an electrically conductive second adhesive provided on the second electrode pattern,
wherein
in a state in which the third constricted section is bent, the first electrode pattern is connected to the first terminal via the first adhesive and the second electrode pattern is connected to the second terminal via the second adhesive.

5. The living body sensor as claimed in claim 3 or 4,
wherein
the battery is set at the battery setting section, the battery being of a coin type, the first terminal of the battery being a positive electrode, and the second terminal of the battery being a negative electrode, and
the second electrode pattern has a circular shape corresponding to a size of a circular shape of the second terminal.

6. The living body sensor as claimed in any one of claims 1-5,
wherein
the body section includes a slit that extends in a direction perpendicular to the longitudinal direction into between the component mounting section and the battery setting section.

7. The living body sensor as claimed in any one of claims 1-6, further comprising:
a plate member attached to the component mounting section to prevent the component mounting section from being warped.

8. The living body sensor as claimed in any one of claims 1-7, further comprising:
a depression switch mounted at the first constricted section side edge of the component mounting section.

9. The living body sensor as claimed in any one of claims 1-8, further comprising:
a housing in which the body section is provided,
wherein
the housing has openings at least at positions corresponding to the first electrode and the second electrode, and
the housing has a surface, the surface being adherable to the living body and being a surface on which the first electrode and the second electrode are exposed.
